# FASCICULE DE BREVET EUROPEEN

(11) **EP 2 409 780 B1**
(45) Date de publication et mention de la délivrance du brevet: **07.05.2014**
(21) Numéro de dépôt: 11174462.9
(22) Date de dépôt: 19.07.2011
(51) Int. Cl.: B05B 17/06, B05B 12/08, A61L 9/14, A61M 11/00, A61M 15/00, G01N 29/14, G01N 29/44, B05B 12/00

(54) **Nébuliseur et procédé de fonctionnement d'un nébuliseur**
Zerstäuber und Betriebsverfahren eines Zerstäubers
Nebuliser and nebuliser operating method

(30) Priorité: 20.07.2010 FR 1055879
(43) Date de publication de la demande: 25.01.2012
(73) Titulaire: System Assistance Medical, 47300 Ledat (FR)
(72) Inventeur: Cinquin, Sébastien, 47150 LA SAUVETAT SUR LEDE (FR)
(74) Mandataire: Zapalowicz, Francis

(56) Documents cités:
- EP-A1- 1 026 482
- EP-A2- 0 849 592
- WO-A1-2006/125251
- WO-A2-2010/067239
- US-A- 4 776 990
- US-A1- 2007 240 712

## Description

La présente invention concerne le domaine des nébuliseurs aptes à générer un brouillard à partir d'un liquide, en particulier un brouillard contenant un médicament, un produit paramédical ou un produit de bien-être, en particulier dans le but que ce brouillard soit inhalé par une personne.

Généralement, les nébuliseurs comprennent une coupelle apte à contenir un liquide à nébuliser et un organe vibrant, en particulier un composant piézo-électrique, placé au-dessous de la coupelle et apte à émettre, sous l'effet d'un signal de commande issu d'un circuit de commande, un faisceau d'ultrasons permettant de transformer le liquide à nébuliser en brouillard. Un liquide tampon, généralement de l'eau, est prévu dans l'espace entre l'organe vibrant et la coupelle, en contact avec cette dernière, ce liquide tampon servant au transfert du faisceau d'ultrasons et constituant une masse thermique permettant d'éviter une élévation de la température du liquide à nébuliser qui pourrait endommager le médicament.

Lorsque l'organe vibrant est en marche et qu'il n'y a plus de liquide à nébuliser dans la coupelle, il y a un risque de détérioration et de perçage de la paroi de la coupelle et un risque d'usure prématurée de l'organe vibrant.

Les documents W02006/125251 et W02010/067239 décrivent des nébuliseurs qui comprennent des organes vibrants produisant des ondes ultrasoniques pour transformer le liquide en brouillard et qui comprennent des capteurs sonores additionnels pour détecter le bruit provoqué par la transformation du liquide en brouillard et délivrer un signal d'avertissement.

Le document US 2007/240712 décrit un nébuliseur qui comprend un organe vibrant produisant une onde ultrasonique pour transformer le liquide en brouillard et dans lequel la variation de la fréquence de résonance produisant l'onde ultrasonique, ou un paramètre électromécanique associé, est mesurée pour déterminer la quantité de liquide restante. Cette variation étant faible, elle est donc difficile à mesurer et n'est pas fiable. Ce nébuliseur peut aussi comprendre un capteur sonore additionnel pour détecter le bruit provoqué par la transformation du liquide en brouillard.

La présente invention a notamment pour but de limiter les inconvénients précités et de proposer une solution particulièrement simple et efficace.

Il est proposé un nébuliseur selon la revendication 1.

Ainsi, l'onde sonore ou le bruit produit lors de la transformation du liquide en brouillard engendre une vibration de l'organe vibrant à des fréquences sonores correspondantes de telle sorte que la détection de telles fréquences sonores peut fournir une information relative au contenu de la coupelle. En particulier, en cas de détection d'absence ou d'inexistence de cette fréquence sonore ou de ce bruit, cela signifie qu'il n'y a plus de liquide à nébuliser dans la coupelle.

Selon l'invention, les moyens de détection comprennent un filtre relié à la liaison entre le circuit de commande et l'organe vibrant et apte à délivrer un signal filtré correspondant à un signal sonore et un moyen d'analyse pour détecter la présence et/ou l'absence de ce signal filtré et/ou la valeur de ce signal filtré par rapport à un seuil (TH) et pour comparer la valeur dudit signal filtré à ce seuil à des instants successifs de comparaison prédéterminés et délivrer un signal d'avertissement lorsque la valeur dudit signal filtré est inférieure ou égale audit seuil un nombre prédéterminé de fois consécutives.

Un avertisseur sonore et/ou lumineux peut être soumis aux moyens de détection afin d'avertir un utilisateur.

II est également proposé un procédé de fonctionnement d'un nébuliseur comprenant une coupelle apte à contenir un liquide et un organe vibrant apte à émettre, sous l'effet d'un signal de commande issu d'un circuit de commande, une onde ultrasonique permettant de transformer le liquide en brouillard, ce procédé consistant à
choisir un organe vibrant apte à détecter une onde sonore,
détecter la présence et/ou l'absence et/ou la valeur d'un signal sonore issu dudit organe vibrant, représentatif de l'onde sonore produite lors de la transformation du liquide en brouillard, en comparant la valeur dudit signal sonore à ce seuil à des instants successifs de comparaison prédéterminés
et délivrer un signal d'avertissement correspondant lorsque la valeur dudit signal sonore est inférieure ou égale audit seuil un nombre prédéterminé de fois consécutives.

Selon une variante, l'activation d'un moyen d'avertissement peut être provoquée en cas d'absence du signal sonore.

Selon une autre variante, la désactivation du circuit de commande peut être provoquée en cas d'absence du signal sonore.

Selon une variante de réalisation, l'organe vibrant peut être apte à émettre une onde ultrasonique dont la fréquence est comprise entre deux et trois mégaHertz et le signal sonore issu dudit organe vibrant, à détecter, peut présenter une fréquence inférieure à cent Hertz.

Un nébuliseur et des modes de fonctionnement de ce dernier vont maintenant être décrits à titre d'exemples non limitatifs, illustrés par le dessin sur lequel :
- la figure 1 représente une coupe verticale partielle d'un nébuliseur ;
- la figure 2 représente schématiquement un circuit électronique associé au nébuliseur de la figure 1 ; et
- la figure 3 représente un graphique amplitude/temps, associé à un mode de fonctionnement du circuit électronique de la figure 2.

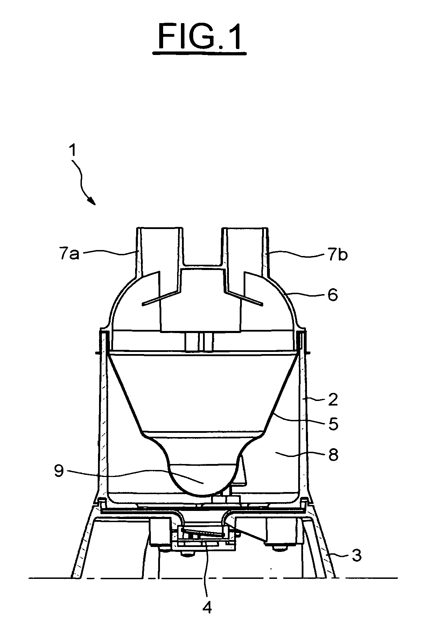

Un nébuliseur 1 illustré sur la figure 1 comprend une cuve 2 portée par un boîtier 3 partiellement représenté, un organe vibrant 4 installé dans le fond de la cuve 2, une coupelle 5 à paroi fine dont le bord périphérique est porté par le bord de la cuve 2 et qui est engagée dans la cuve 2 de telle sorte que le fond de cette coupelle 4 est à distance au-dessus du fond de la cuve 2 et de l'organe vibrant 4, ainsi qu'un couvercle 6 également porté par le bord de la cuve 2 et présentant des embouchures 7a et 7b.

L'espace 8 entre la cuve 2 et la coupelle 5 est, au moins partiellement, rempli d'un liquide tel que de l'eau, de telle sorte qu'au moins la partie inférieure de la coupelle 5 plonge dans ce liquide.

Dans la coupelle 5 peut être versé un liquide 9 à nébuliser, par exemple un liquide contenant un médicament, un produit paramédical ou un produit de bien-être.

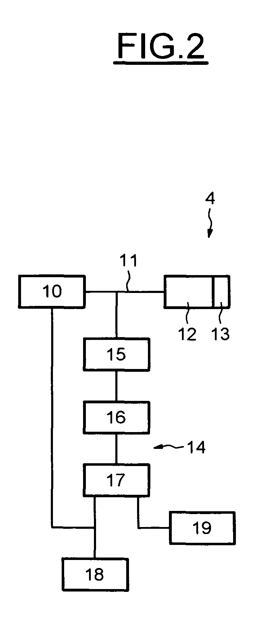

Comme illustré sur la figure 2, l'organe vibrant 3 est relié à un circuit électronique de commande 10 par une liaison 11 par exemple filaire, installé dans le boîtier 3.

Sous l'effet d'un signal électrique issu de l'organe électronique de commande 10, l'organe vibrant 4 émet un faisceau d'ultrasons ou une onde ultrasonique qui se propage dans l'eau contenue dans l'espace 8, puis au travers de la paroi de la coupelle 5, puis au travers du liquide 9 à nébuliser. Sous l'effet de cette onde, il se produit, localement à la surface du liquide 9, un jet de telle sorte qu'un éclatement du liquide en particules permet de transformer progressivement le liquide 9 à nébuliser en brouillard, la taille des particules dépendant de la longueur d'onde de l'onde émise par l'organe vibrant 4.

Le brouillard généré peut être inhalé par un utilisateur grâce à des tuyaux qui peuvent être branchés sur les embouchures 7a et 7b, dans le but de faire circuler de l'air au-dessus du liquide 9 et dans le couvercle 6 pour emporter le brouillard.

Selon un exemple, l'organe vibrant 4 comprend un oscillateur ultrasonique 12 relié à un organe piézo-céramique 13. La fréquence de l'onde émise par l'organe piézo-céramique 13 peut être d'environ 2,4 MégaHertz.

Le nébuliseur 1 comprend en outre des moyens de détection 14 pour détecter la présence et/ou l'absence de liquide 9 à nébuliser dans la coupelle 4 et en avertir l'utilisateur. Ces moyens de détection 14 sont basés sur une détection de la présence ou de l'absence du bruit produit lors de la transformation du liquide 9 à nébuliser en brouillard.

Comme illustré sur la figure 2, les moyens de détection 14 comprennent un capteur sonore qui est constitué directement de l'organe vibrant 4. L'organe piézo-céramique 13 de l'organe vibrant 4 peut en effet être sensible à l'onde sonore produite lors de la transformation du liquide 8 à nébuliser en brouillard, de telle sorte que l'organe vibrant 4 peut délivrer sur la liaison 11 un signal sonore, c'est-à-dire un signal électrique fréquentiel correspondant à une fréquence sonore.

Les moyens de détection 14 comprennent en outre une chaîne de détection qui comprend un filtre 15 relié à la liaison 11, permettant de filtrer le signal sonore, par exemple ayant une fréquence inférieure à 100 Hertz, puis un circuit 16 d'amplification et de mise en forme, par moyennage ou intégration, du signal issu du filtre 15, permettant de délivrer un signal de tension Sc correspondant au signal sonore.

Ce signal Sc est alors dirigé vers un circuit d'analyse 17 apte à analyser ce signal Sc selon un programme et à délivrer un signal d'activation Sa pour activer un avertisseur sonore et/ou lumineux 18 en fonction de la valeur du signal Sc et/ou pour désactiver le circuit électronique de commande 10. Le circuit d'analyse 17 et l'avertisseur sonore et/ou lumineux 18 sont portés par le boîtier 3 et ne sont pas représentés sur la figure 1.

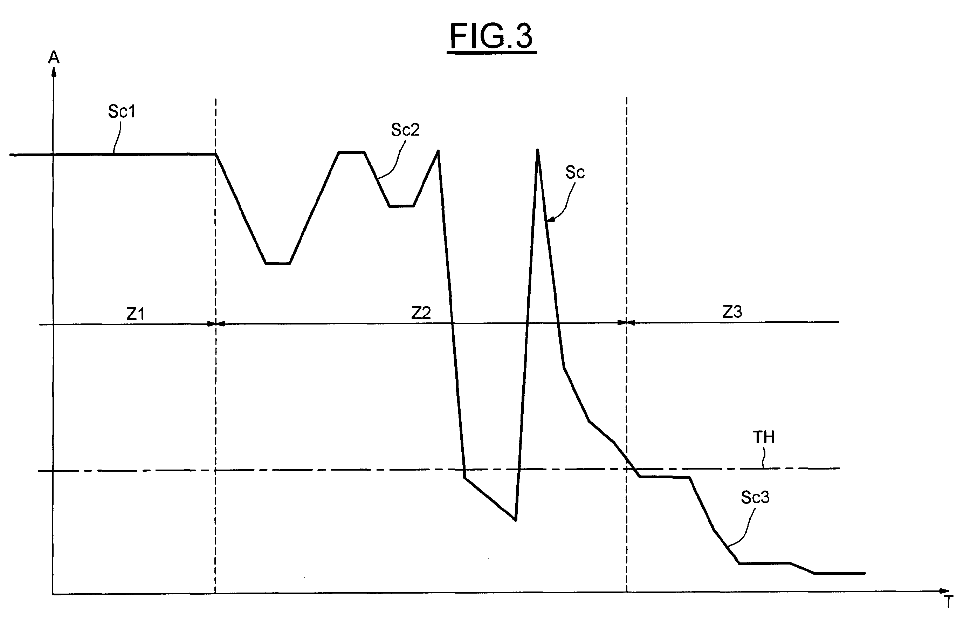

La figure 3 illustre un mode d'analyse de la valeur ou l'amplitude du signal Sc par rapport à la valeur d'un seuil TH, effectué par le circuit d'analyse 17.

On suppose avoir déposé une certaine quantité de liquide 9 à nébuliser dans la coupelle 5 et avoir mis en marche le nébuliseur 1 pour produire du brouillard.

Dans une zone temporelle Z1, tant que la coupelle 5 contient suffisamment de liquide 9 à nébuliser, le bruit produit par la nébulisation est relativement fort. En conséquence, la valeur Sc1 du signal Sc est et reste largement supérieure à la valeur du seuil TH. La valeur du seuil TH peut être comprise entre 10% et 30% de la valeur du signal Sc dans ce cas.

Puis, dans une zone temporelle Z2 consécutive, quand la liquide vient à manquer et qu'il ne reste plus dans la coupelle 5 que quelques gouttes du liquide 9 à nébuliser, le bruit produit par la nébulisation se réduit et devient haché. En conséquence, la valeur Sc2 du signal Sc effectue des descentes et des montées et peut passer, au cours de brèves durées, sous la valeur du seuil TH.

Puis, dans une zone temporelle Z3 consécutive, quand la coupelle 5 devient quasiment vide ou vide, le bruit produit par la nébulisation devient très faible puis inexistant. En conséquence, la valeur Sc3 du signal Sc devient et reste inférieure à la valeur du seuil TH.

Pour analyser les circonstances décrites ci-dessus, le circuit d'analyse 17 peut être programmé pour comparer la valeur du signal Sc à la valeur du seuil TH à des instants successifs de comparaison prédéterminés selon par exemple la disposition suivante.

Si, lors d'une comparaison, la valeur du signal Sc est supérieure à la valeur du seuil TH lors d'un instant de comparaison, le circuit d'analyse 17 effectuera une nouvelle comparaison au bout d'une durée longue prédéterminée D1, par exemple égale à trois minutes.

Si, lors d'une comparaison, la valeur du signal Sc est inférieure ou égale à la valeur du seuil TH, le circuit d'analyse 17 effectuera une nouvelle comparaison au bout d'une durée courte prédéterminée D2, par exemple égale à dix secondes.

Le circuit d'analyse 17 est programmé pour être apte à compter le nombre de fois consécutives pour lesquelles la valeur du signal Sc est inférieure ou égale à la valeur du seuil TH et est programmé pour délivrer un signal d'avertissement Ss dès que ce nombre atteint une valeur prédéterminée Np, par exemple 200 fois consécutives.

Ainsi, les zones Z1 et Z2 sont détectées par le fait que le circuit d'analyse 17 effectue des comparaisons espacées de la durée longue prédéterminée D1 ou que la valeur prédéterminée Np du nombre de fois consécutives pour lesquelles la valeur du signal Sc est inférieure ou égale à la valeur du seuil TH n'est pas atteinte.

Par contre, la zone Z3 est détectée par le fait que le circuit d'analyse 17 a effectué un nombre de fois consécutives de comparaisons pour lesquelles la valeur du signal Sc est inférieure ou égale à la valeur du seuil TH, tel que la valeur prédéterminée Np est atteinte. Alors, le circuit d'analyse 17 génère le signal d'activation Sa.

Dans une variante, le signal d'activation Sa active l'avertisseur sonore et/ou lumineux 18 pour que l'utilisateur redépose du liquide 9 à nébuliser dans la coupelle 5 ou désactive le circuit électronique de commande 10 de telle sorte que l'organe vibrant 4 cesse d'être activé.

Dans une autre variante, le circuit d'analyse 17 génère le signal d'activation Sa pour désactiver le circuit électronique de commande 10 de telle sorte que l'organe vibrant 4 cesse d'être activé automatiquement, sa remise en marche ne pouvant se produire que si du liquide 9 à nébuliser est redéposé dans la coupelle 5.

Les deux variantes ci-dessus peuvent être naturellement être mise en oeuvre conjointement.

Il résulte de ce qui précède que la coupelle 5 peut être protégée contre un éventuel endommagement ou une éventuelle perforation en cas d'absence de liquide 9 à nébuliser dans la coupelle 5.

Selon une variante, le circuit d'analyse 17 pourrait être programmé pour délivrer le signal d'avertissement Sa, ou un signal distinct, pour commander l'avertisseur sonore et/ou lumineux 18, distinctement, ou un avertisseur distinct, dans le cas où le nombre de fois consécutives pour lesquelles la valeur du signal Sc est inférieure ou égale à la valeur du seuil TH atteint une valeur déterminée inférieure à la valeur Np, pour par exemple signaler à l'utilisateur qu'il ne reste que quelques gouttes du liquide 9 à nébuliser dans la coupelle 5.

Selon une autre variante, également représentée sur la figure 2, la valeur du signal Sc, dans la zone précitée Z1, peut être représentative de la quantité de liquide 9 à nébuliser contenue dans la coupelle 5. Dans ce cas, le circuit d'analyse 17 pourrait délivrer un signal Sb relatif à cette quantité pour fournir une information correspondante à l'utilisateur par exemple par l'intermédiaire d'un afficheur 19.

La présente invention ne se limite pas aux exemples ci-dessus décrits. Bien d'autres variantes de réalisation sont possibles, sans sortir du cadre défini par les revendications annexées.

## Revendications

1. Nébuliseur comprenant une coupelle (5) apte à contenir un liquide et un organe vibrant (4) apte à émettre, sous l'effet d'un signal de commande issu d'un circuit de commande (10), un faisceau ultrasonique permettant de transformer le liquide en brouillard, comprenant en outre des moyens de détection (14) du bruit produit par la nébulisation pour détecter la présence et/ou l'absence d'un signal sonore issu dudit organe vibrant et/ou déterminer la valeur d'un tel signal sonore, ou d'un signal correspondant à un signal sonore issu dudit organe vibrant,
**caractérisé en ce que** les moyens de détection (14) comprennent un filtre relié à la liaison entre le circuit de commande (10) et l'organe vibrant (4) et apte à délivrer un signal filtré correspondant à un signal sonore et un moyen d'analyse (17) pour détecter la présence et/ou l'absence de ce signal filtré et/ou la valeur de ce signal filtré par rapport à un seuil (TH) et pour comparer la valeur dudit signal filtré à ce seuil à des instants successifs de comparaison prédéterminés et délivrer un signal d'avertissement lorsque la valeur dudit signal filtré est inférieure ou égale audit seuil un nombre prédéterminé de fois consécutives.

2. Nébuliseur selon la revendication 1, comprenant un avertisseur sonore et/ou lumineux (18) soumis aux moyens de détection (17).

3. Nébuliseur selon l'une des revendications précédentes, dans lequel l'organe vibrant (4) est placé au-dessous de la coupelle (5).

4. Nébuliseur selon l'une quelconque des revendications précédentes, dans lequel l'organe vibrant (4) est un composant piézocéramique.

5. Procédé de fonctionnement d'un nébuliseur comprenant une coupelle (5) apte à contenir un liquide et un organe vibrant (4) apte à émettre, sous l'effet d'un signal de commande issu d'un circuit de commande (10), une onde ultrasonique permettant de transformer le liquide en brouillard, comprenant :
choisir un organe vibrant apte à détecter une onde sonore,
détecter la présence et/ou l'absence et/ou la valeur d'un signal sonore issu dudit organe vibrant, représentatif de l'onde sonore produite lors de la transformation du liquide en brouillard, par rapport à un seuil, en comparant la valeur dudit signal sonore à ce seuil à des instants successifs de comparaison prédéterminés,
et délivrer un signal d'avertissement lorsque la valeur dudit signal sonore est inférieure ou égale audit seuil un nombre prédéterminé de fois consécutives.

6. Procédé selon la revendication 5, comprenant l'activation d'un moyen d'avertissement en cas d'absence du signal sonore.

7. Procédé selon l'une des revendications 5 et 6, comprenant la désactivation du circuit de commande en cas d'absence du signal sonore.

8. Procédé selon l'une quelconque des revendications 5 à 7, dans lequel l'organe vibrant (4) est apte à émettre une onde ultrasonique dont la fréquence est comprise entre deux et trois mégaHertz et dans lequel le signal sonore issu dudit organe vibrant, à détecter, présente une fréquence inférieure à cent Hertz.

## Patentansprüche

1. Zerstäuber, umfassend eine Schale (5), die geeignet ist, eine Flüssigkeit zu enthalten, und ein vibrierendes Element (4), das geeignet ist, unter der Wirkung eines von einer Steuerschaltung (10) kommenden Steuersignals einen Ultraschallstrahl zu entsenden, der es ermöglicht, die Flüssigkeit in einen Nebel umzuwandeln, ferner umfassend Mittel (14) zur Erfassung des Geräuschs, der durch die Zerstäubung erzeugt wird, um das Vorliegen und/oder Fehlen eines Tonsignals, das von dem vibrierenden Element stammt, zu erfassen und/oder den Wert eines solchen Tonsignals oder eines Signals entsprechend einem von dem vibrierenden Element stammenden Tonsignal zu bestimmen, **dadurch gekennzeichnet, dass** die Erfassungsmittel (14) einen Filter, der mit der Verbindung zwischen der Steuerschaltung (10) und dem vibrierenden Element (4) verbunden und geeignet ist, ein gefiltertes Signal zu liefern, das einem Tonsignal entspricht, und ein Analysemittel (17) umfassen, um das Vorliegen und/oder Fehlen dieses gefilterten Signals und/oder den Wert dieses gefilterten Signals in Bezug zu einer Schwelle (TH) zu erfassen, und um den Wert des gefilterten Signals mit dieser Schwelle zu vorbestimmten aufeinanderfolgenden Vergleichsmomenten zu vergleichen und ein Warnsignal zu liefern, wenn der Wert des gefilterten Signals eine vorbestimmte Anzahl von aufeinanderfolgenden Malen kleiner oder gleich der Schwelle ist,

2. Zerstäuber nach Anspruch 1, umfassend einen Ton- und/oder Lichtwarnsender (18), der den Erfassungsmitteln (14) untergeordnet ist.

3. Zerstäuber nach einem der vorhergehenden Ansprüche, bei dem das vibrierende Element (4) unter der Schale (5) angeordnet ist.

4. Zerstäuber nach einem der vorhergehenden Ansprüche, bei dem das vibrierende Element (4) eine piezokeramische Komponente ist.

5. Betriebsverfahren eines Zerstäubers, umfassend eine Schale (5), die geeignet ist, eine Flüssigkeit zu enthalten, und ein vibrierendes Element (4), das geeignet ist, unter der Wirkung eines von einer Steuerschaltung (10) kommenden Steuersignals eine Ultraschallwelle zu entsenden, die es gestattet, die Flüssigkeit in Nebel umzuwandeln, umfassend:
Auswahl eines vibrierenden Elements, das geeignet ist, eine Schallwelle zu erfassen,
Erfassen des Vorliegens und/oder Fehlens und/oder des Werts eines Tonsignals, das von dem vibrierenden Element stammt und für die Schallwelle repräsentativ ist, die bei der Umwandlung der Flüssigkeit in Nebel erzeugt wird, in Bezug auf eine Schwelle, wobei der Wert des Tonsignals mit dieser Schwelle zu vorbestimmten aufeinanderfolgenden Vergleichsmomenten verglichen wird,
und Lieferung eines Warnsignals, wenn der Wert des Tonsignals eine vorbestimmte Anzahl von aufeinanderfolgenden Malen kleiner oder gleich der Schwelle ist.

6. Verfahren nach Anspruch 5, umfassend die Aktivierung eines Warnmittels bei Fehlen des Tonsignals.

7. Verfahren nach einem der Ansprüche 5 und 6, umfassend die Deaktivierung der Steuerschaltung bei Fehlen des Tonsignals.

8. Verfahren nach einem der Ansprüche 5 bis 7, bei dem das vibrierende Element (4) geeignet ist, eine Ultraschallwelle zu entsenden, deren Frequenz zwischen zwei und drei Megahertz beträgt, und bei dem das zu erfassende, von dem vibrierenden Element stammende Tonsignal eine Frequenz unter hundert Hertz aufweist.

## Claims

1. Nebulizer comprising a cupel (5) capable of containing a liquid and a vibrating member (4) capable of emitting, under the effect of a control signal originating from a control circuit (10), an ultrasound beam making it possible to transform the liquid into mist, further comprising detection means (14) for detecting the noise produced by the nebulization in order to detect the presence and/or the absence of a sound signal originating from said vibrating member and/or for determining the value of such a sound signal, or of a signal corresponding to a sound signal originating from said vibrating member, **characterized in that** the detection means (14) comprise a filter linked to the connection between the control circuit (10) and the vibrating member (4) and capable of delivering a filtered signal corresponding to a sound signal and an analysis means (17) for detecting the presence and/or the absence of this filtered signal and/or the value of this filtered signal relative to a threshold (TH) and for comparing the value of said filtered signal with this threshold at predetermined successive comparison times and delivering a warning signal when the value of said filtered signal is less than or equal to said threshold a predetermined number of consecutive times.

2. Nebulizer according to Claim 1, comprising a sound and/or light warning element (18) subjected to the detection means (14).

3. Nebulizer according to either of the preceding claims, in which the vibrating member (4) is placed beneath the cupel (5).

4. Nebulizer according to any one of the preceding claims, in which the vibrating member (4) is a piezoceramic component.

5. Method for operating a nebulizer comprising a cupel (5) capable of containing a liquid and a vibrating member (4) capable of emitting, under the effect of a control signal originating from a control circuit (10), an ultrasound wave making it possible to transform the liquid into mist, comprising:
choosing a vibrating member capable of detecting a sound wave,
detecting the presence and/or the absence and/or the value of a sound signal originating from said vibrating member, representative of the sound wave produced during the transformation of the liquid into mist, relative to a threshold, by comparing the value of said sound signal with this threshold at predetermined successive comparison times,
and delivering a corresponding warning signal when the value of said sound signal is less than or equal to said threshold a predetermined number of consecutive times.

6. Method according to Claim 5, comprising the activation of a warning means in the event of absence of the sound signal.

7. Method according to either of Claims 5 and 6, comprising the deactivation of the control circuit in the event of absence of the sound signal.

8. Method according to any one of Claims 5 to 7, in which the vibrating member (4) is capable of emitting an ultrasound wave of which the frequency is between two and three megaHertz and in which the sound signal to be detected, that originates from said vibrating member, has a frequency of less than one hundred Hertz.
